# FASCICULE DE BREVET EUROPEEN

(11) **EP 1 004 255 B1**
(45) Date de publication et mention de la délivrance du brevet: **12.09.2001**
(21) Numéro de dépôt: 99402741.5
(22) Date de dépôt: 04.11.1999
(51) Int. Cl.: A46D 9/06, D06M 15/333, A45D 34/04, A61K 7/043

(54) **Pinceau de vernis a ongles et ensemble d'application de vernis a ongles muni d'un tel pinceau**
Nagellackpinsel und Gebinde mit einem solchen Pinsel
Nail varnish brush and application package comprising such a brush

(30) Priorité: 27.11.1998 FR 9814986
(43) Date de publication de la demande: 31.05.2000
(73) Titulaire: L'OREAL, 75008 Paris (FR)
(72) Inventeur: Ramin, Roland, 75014 Paris (FR); Lacoutiere, Stéphane, 75015 Paris (FR)
(74) Mandataire: Kromer, Christophe

(56) Documents cités:
- EP-A- 0 556 081
- WO-A-97/22666
- FR-A- 2 685 925
- GB-A- 568 150
- US-A- 2 665 443
- US-A- 4 126 144
- US-A- 4 192 649
- US-A- 4 311 695
- US-A- 5 354 339
- PATENT ABSTRACTS OF JAPAN vol. 098, no. 009, 31 juillet 1998 (1998-07-31) & JP 10 110387 A (TORAY IND INC), 28 avril 1998 (1998-04-28)
- DATABASE WPI Week 8824 Derwent Publications Ltd., London, GB; AN 88165901 XP002112690 & JP 63 105189 A (KANEGAFUCHI CHEM KK), 10 mai 1988 (1988-05-10)

## Description

La présente invention se rapporte à un pinceau pour l'application d'un produit de maquillage ou de soin tel qu'un vernis ou un soin des ongles, un fard liquide pour les lèvres, ou un fond de teint liquide, du genre de ceux qui comprennent des poils disposés sensiblement parallèlement les uns aux autres en touffe, et fixés sur un support appelé tige de pinceau.

Les vernis à ongles sont généralement conditionnés dans un ensemble d'application constitué d'un récipient muni d'un goulot et d'un bouchon solidaire d'un pinceau d'application immergé, en position de stockage, dans le produit de vernis à ongles contenu dans le récipient.

Pour une bonne application du vernis à ongles, il est important que la touffe de poils du pinceau soit uniforme afin de déposer une couche régulière de vernis sur les ongles. Or, lors du transport et du stockage du pinceau avant son conditionnement avec le récipient contenant le produit de vernis, la touffe de poils peut être déformée, par exemple par pliage ou recourbement d'une ou de plusieurs poils de la touffe : les poils sont alors écartés et ne forment plus une touffe homogène. Une telle touffe déformée ne permet pas une application régulière du produit sur les ongles ; de plus, le pinceau est difficile à introduire à travers le goulot du récipient, nuisant ainsi au bon déroulement du processus industriel de conditionnement du vernis.

Pour éviter la déformation de la touffe de poils, il est possible de traiter la touffe de poils, avant son conditionnement dans le récipient, par imprégnation avec une solution de polymère filmogène, dite solution renforcatrice. Après séchage, un dépôt du polymère filmogène se trouve en surface des poils, empêchant ces derniers de se déformer ou de s'écarter : les poils sont ainsi renforcés.

La solution renforcatrice doit être suffisamment fluide pour permettre une imprégnation rapide de la touffe de poils. La solution doit être apte à imprégner une partie suffisante de la touffe par capillarité pour assurer un bon maintien de la touffe. La solution après dépôt sur la touffe doit sécher rapidement pour laisser sur la touffe un dépôt de polymère non collant, ne s'effritant pas, non rigide et non cassant. Le dépôt de polymère doit pouvoir se dissoudre rapidement lorsque la touffe est immergée dans le produit de vernis à ongles, sans perturber les propriétés cosmétiques du produit, ni le déstabiliser.

La nitrocellulose est un des polymères filmogènes les plus couramment utilisés dans les vernis à ongles en milieu solvant organique. Pour ces vernis, la solution renforcatrice est une solution de nitrocellulose et d'agent plastifiant. Il est aussi connu des vernis à ongles en milieu aqueux comprenant des particules de polymères filmogènes dispersées dans le milieu aqueux, comme décrit par exemple dans le document EP-A-648485 ; dans ces produits, le polymère filmogène à l'état de particules dispersées est insoluble dans l'eau. Pour traiter les pinceaux destinés à l'application de vernis à l'eau, il est possible d'employer comme solution renforcatrice une solution aqueuse contenant un polymère hydrosoluble. Ce polymère hydrosoluble est de nature différente du polymère filmogène insoluble dans l'eau présent dans le produit de vernis. Contrairement aux vernis en milieu solvant comprenant de la nitrocellulose, il est difficile de traiter la touffe du pinceau avec le même polymère filmogène présent dans le produit de vernis.

Le document GB-A-568150 décrit l'enrobage des poils de polyamide par un polymère d'oxyde d'oléfine pour les rendre moins glissants pour la fabrication de brosses telles que les brosses à dents ou les brosses pour cheveux. Les poils sont enrobés avant leur fixation en touffe sur la brosse.

Le document US-A-5,354,339 décrit l'empesage de tissus, destinés aux travaux de couture et de broderie, avec une solution aqueuse d'alcool polyvinylique.

Il est connu du document US-A-4,126,144 un ensemble d'application de vernis à ongles muni d'un pinceau et d'un réservoir contenant une composition comprenant une dispersion aqueuse de polymère latex qui est stabilisée par addition d'alcool polyvinylique partiellement acétylé.

Le but de la présente invention est donc de proposer un pinceau renforcé convenant pour l'application de vernis à ongles à milieu aqueux.

De façon surprenante, la Demanderesse a constaté qu'un tel pinceau pouvait être obtenu en employant un alcool polyvinylique particulier pour renforcer la touffe de poils. Ce polymère se dissout facilement dans l'eau et permet d'obtenir une solution aqueuse fluide permettant une imprégnation rapide de la touffe ; la solution aqueuse déposée sur la touffe sèche rapidement et laisse sur les poils un dépôt non collant, ne s'effritant pas. Les poils ainsi traités restent suffisamment souples tout en ne s'écartant pas et forment une touffe homogène, même après les contraintes de transport et de stockage du pinceau avant conditionnement. De plus, après le conditionnement du pinceau sur le récipient de vernis, le dépôt de polymère se dissout rapidement dans le produit de vernis contenant de l'eau, sans modifier les propriétés cosmétiques du vernis, ni déstabiliser le produit.

De façon plus précise, l'invention a pour objet un pinceau pour l'application d'un produit de maquillage ou de soin, comportant une touffe de poils sensiblement parallèles, une première extrémité de ces poils étant fixée à une extrémité libre d'une tige, caractérisé par le fait que les poils de la touffe sont imprégnés en surface d'un alcool polyvinylique partiellement acétylé comportant au moins des unités de formules (I) et (II) : 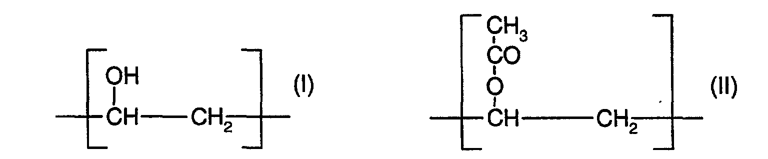 dans lesquelles les unités de formule (II) sont présentes dans des proportions allant de 3 % à 40 % en mole par rapport au polymère, ledit alcool polyvinylique ayant un poids moléculaire moyen en poids allant de 10.000 à 190.000.

L'invention a encore pour objet un procédé de gainage de poils d'une touffe d'un pinceau pour l'application d'un produit de maquillage, comportant une touffe de poils sensiblement parallèles, une première extrémité de ces poils étant fixée à une extrémité libre d'une tige, caractérisée par le fait que l'on met en contact au moins une partie de la touffe de poils avec une composition aqueuse comprenant un alcool polyvinylique tel que défini précédemment, puis on retire la touffe de poils de la composition et on la laisse sécher.

L'invention a aussi pour objet un pinceau pour l'application d'un produit de maquillage susceptible d'être obtenu selon le procédé défini précédemment.

L'invention a encore pour objet un ensemble d'application de produit de vernis ou de soin des ongles constitué d'un récipient muni d'un goulot et d'un bouchon solidaire d'un pinceau tel que défini précédemment, immergé, en position de stockage, dans un produit de vernis ou de soin des ongles comprenant un milieu aqueux contenu dans ce récipient.

L'invention a également pour objet un ensemble d'application de produit de vernis ou de soin des ongles constitué d'un récipient muni d'un goulot et d'un bouchon solidaire d'un pinceau, immergé, en position de stockage, dans un produit de vernis ou de soin des ongles contenu dans ce récipient, caractérisé par le fait que ledit produit comprend un milieu aqueux contenant au moins un alcool polyvinylique tel que défini précédemment.

L'alcool polyvinylique utilisé selon l'invention a un poids moléculaire moyen en poids allant de 10.000 à 190.000, de préférence de 10.000 à 120.000, et mieux de 31.000 à 50.000, de façon à obtenir une solution renforcatrice suffisamment fluide pour pouvoir imprégner par capillarité la touffe de poils du pinceau.

Avantageusement, les unités de formule (II) peuvent être présentes dans l'alcool polyvinylique en une proportion allant de 10 à 15 % en mole.

Dans un mode préféré de réalisation de l'invention, l'alcool polyvinylique consiste essentiellement en des unités de formules (I) et (II).

Comme alcool polyvinylique utilisable selon l'invention, on peut utiliser ceux vendus sous les dénominations "AIRVOL 203", "AIRVOL 205", "AIRVOL 6108", "AIRVOL 523", "AIRVOL 540" par la société AIR PRODUCTS CHEMICAL, "RHODOVIOL 4/125", "RHODOVIOL 14/135", "RHODOVIOL 25/140" par la société RHÔNE POULENC, "MOWIOL 4-88", "MOWIOL 40-88", "MOWIOL 18-88" par la société HOECHST, "ELVANOL 51-05", "ELVANOL 52-22" par la société DUPONT.

Les poils de la touffe peuvent être en un matériau choisi parmi les polyamides, les polyesters, les polyéther-bloc-amides, le polyéthylène, le polytétrafluoroéthylène, le polyfluorure de vinylidène, les polychlorures de vinyle, la viscose, la rayonne, les polyacétals, les soies naturelles, ou leurs mélanges.

Les poils constituant le pinceau peuvent avoir, de préférence, une section inscrite dans un cercle (φ) de diamètre allant par exemple de 4/100 à 40/100 de mm. Selon une variante de réalisation de l'invention, la touffe peut comprendre un mélange de poils de petite section, ou petits poils, et de poils de section transversale plus importante ou gros poils, la proportion de gros poils pouvant aller de 2 % à 95 % en volume, par rapport au volume total de la touffe du pinceau, et de préférence de 10 % à 90 %. En particulier, les petits poils peuvent avoir une section inscrite allant de 4/100 à 10/100 de mm et les gros poils peuvent avoir une section inscrite allant de 11/100 à 40/100 de mm.

Avantageusement, la touffe des poils du pinceau peut avoir une longueur allant de 5 à 25 mm et de préférence, une longueur allant de 13 à 20 mm.

Selon un aspect intéressant de l'invention, les poils présentent une première extrémité obtenue en pliant en U un faisceau de fibres, la base de U étant maintenue par une agrafe enfoncée dans un logement pratiqué dans l'extrémité libre de la tige du pinceau. Avantageusement, ce logement peut être un cylindre de révolution. On peut envisager également de le conformer sous forme ovale, allongée, cruciforme, ou en forme de tuile demi-ronde. Ce logement peut être évasé vers l'extrémité libre de la tige. La fixation de la touffe de poils dans ce logement peut être effectuée également par collage ou par tout autre moyen utilisé habituellement pour la fabrication de pinceaux, par exemple par une virole.

Le nombre de poils constituant la touffe peut avantageusement aller de 100 à 600.

La matière constituant les poils peut avantageusement contenir un agent modifiant leur état de surface et/ou leurs caractéristiques de glissement et/ou réduisant leur mouillabilité à l'eau et/ou au solvant contenu dans le produit de maquillage et notamment dans le vernis, ou bien un agent anti-statique.

Avantageusement, l'agent améliorant la caractéristique de glissement des poils et réduisant leur mouillabilité à l'eau, peut être incorporé dans la matière des poils selon un taux allant de 0,2 % à 15 % en poids.

Cet agent de glissement peut être choisi de préférence dans le groupe formé par le polytétrafluoroéthylène, le nitrure de bore, le bisulfure de molybdène, le graphite, les silicones, les fullerènes, le talc.

Avantageusement, une partie des poils au moins, peut présenter des légères ondulations sur leur longueur. Les poils peuvent avoir des sections transversales dont la forme est choisie dans le groupe des formes circulaires, annulaires polygonales cruciformes, rectangulaires, polylobes, en U, en C, et en V, et des formes comportant au moins une rainure capillaire.

L'extrémité libre des poils constituant le pinceau peut être agencée en forme de tête d'épingle, notamment obtenue par traitement thermique, par exemple par flammage. L'extrémité libre des poils peut également être effilée, cet effilement étant obtenu par exemple par meulage ou par cardage.

La section transversale du pinceau peut avoir différentes formes ; notamment, la touffe peut avoir une section circulaire, une section en tuile demi-ronde, une section ovale ou cruciforme. Par ailleurs, les poils peuvent avoir des longueurs différentes. En particulier, l'extrémité libre du pinceau peut être plate ou arrondie.

Des pinceaux utilisables selon l'invention sont notamment décrits dans les documents EP-A-556081, EP-A-581922, EP-A-651955 et EP-A-673612, dont le contenu est incorporé dans la présente demande à titre de référence.

Pour obtenir le pinceau selon l'invention, on met en contact au moins une partie, voire la totalité, de la touffe de poils du pinceau avec une composition aqueuse comprenant l'alcool polyvinylique tel que défini précédemment. Avantageusement, on peut immerger dans la composition la moitié de la longueur de la touffe de poils ; on peut également effleurer l'extrémité de la touffe avec la composition pendant un temps suffisant pour que la touffe s'imprègne par capillarité. Le temps de contact peut être relativement court, et notamment inférieur ou égal à 5 secondes ; on peut même procéder à un trempage instantané.

L'alcool polyvinylique se dissolvant parfaitement dans l'eau, la composition aqueuse utilisée dans le procédé selon l'invention, dite solution renforcatrice, se présente sous la forme d'une solution aqueuse dudit alcool polyvinylique.

De préférence, l'alcool polyvinylique peut être présent dans la composition en une teneur allant de 0,1 % à 10 % en poids, et de préférence de 0,5 % à 5 % en poids, par rapport au poids total de la composition, pour permettre d'obtenir une solution bien fluide favorisant une bonne imprégnation de la touffe de poils.

Après l'imprégnation, le pinceau est retiré de la solution renforcatrice puis laissé séché jusqu'à ce que le dépôt de polymère déposé sur les poils soit sec. Le séchage peut être effectué à l'air ambiant ou bien à l'aide d'une source de chaleur ou d'air pulsé (ventilateur).

Pour améliorer le temps de séchage de la solution renforcatrice après imprégnation de la touffe, la solution renforcatrice peut comprendre en outre au moins un alcool inférieur en C₂-C₅, et en particulier l'éthanol. La teneur en alcool inférieur dans la composition peut aller de 0 % à 80 % en poids, par rapport au poids total de la composition, et de préférence de 40 % à 70 % en poids.

La solution renforcatrice peut comprendre en outre un agent de mouillage pour diminuer la tension superficielle de la solution et pour favoriser une bonne imprégnation des poils du pinceau. Comme agent de mouillage, on peut par exemple employer un diméthicone copolyol. L'agent de mouillage peut être notamment présent en une teneur allant de 0,1 % à 15 % en poids, par rapport au poids total d'alcool polyvinylique, et de préférence de 0,5 % à 5 % en poids.

Le diméthicone copolyol peut être choisi parmi les composés de formule générale (III) : 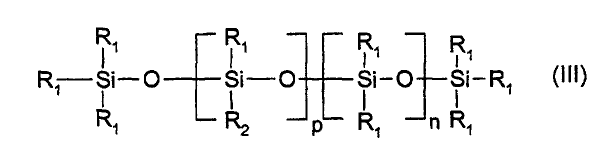 formule dans laquelle :
- R₁, identiques ou différents, représentent un atome d'hydrogène, un radical alkyle linéaire ou ramifié en C₁-C₃₀ ou un radical phényle,
- R₂, identiques ou différents, représentent -(CₓH₂ₓ)-(OC₂H₄)ₐ-(OC₃H₆)_{b}-OR₃,
- R₃, identiques ou différents, sont choisis parmi un atome d'hydrogène, un radical alkyle, linéaire ou ramifié, ayant de 1 à 12 atomes de carbone, un radical acyle, linéaire ou ramifié ayant de 2 à 12 atomes de carbone,
- n varie de 0 à 1000,
- p varie de 1 à 30,
- a varie de 0 à 50,
- b varie de 0 à 50,
- a + b est supérieur ou égal à 1,
- x varie de 1 à 5,
- le poids moléculaire moyen en nombre étant supérieur ou égal à 15000 et de préférence compris entre 25000 et 75000.

De façon préférentielle, on utilise les silicones oxyalkylénées de formule générale (III) qui répondent à au moins une des, et de préférence à toutes les, conditions suivantes :
- R₁ désigne le radical méthyle.
- R₃ représente un atome d'hydrogène, un radical méthyle ou un radical acétyle, et de préférence hydrogène.
- p varie de 8 à 20.
- a est compris entre 5 et 40 et de préférence entre 15 et 30.
- b est compris entre 5 et 40 et de préférence entre 15 et 30.
- x est égal à 2 ou 3.
- n varie de 20 à 600, de préférence de 50 à 500 et encore plus particulièrement de 100 à 300.
De telles silicones sont par exemple décrites dans le brevet US-4,311,695 qui est inclus à titre de référence.

Des diméthicones copolyols ont en particulier été présentés par la société DOW CORNING lors du 17ème congrès international de l'I.F.S.C.C. d'octobre 1992 et rapportés dans l'article "Water-soluble dimethicone copolyol waxes for personal care industry" de Linda Madore et al., pages 1 à 3.
Ces diméthicones copolyols sont des polydiméthylsiloxanes (PDMS) comportant une ou plusieurs fonctions éthers, solubles dans l'eau (oxyalkylène, notamment oxyéthylène et/ou oxypropylène).
De tels diméthicones copolyols sont notamment vendus par la société GOLDSCHMIDT sous la dénomination ABIL B8851 ou ABIL B88183. On peut citer aussi les composés KF 351 à 354 et KF 615 A vendus par la société SHIN ETSU ou la DMC 6038 de la société WACKER.
Les dérivés de diméthicones copolyols utilisables peuvent être en particulier les diméthicones copolyols à groupement phosphate, sulfate, chlorure de myristamide propyldiméthylammonium, stéarate, amine, glycomodifié, etc. On peut utiliser comme dérivés de diméthicones copolyols notamment les composés vendus par la société SILTECH sous la dénomination Silphos A100, Siltech amine 65, Silwax WDIS, myristamido silicone quat, ou par la société PHOENIX sous la dénomination Pecosil PS 100.
On peut également utiliser les dérivés vendus par la société WACKER sous la dénomination VP 1661, ou par la société DOW CORNING sous la dénomination 2501 cosmetic wax. On peut encore utiliser les dérivés vendus par la société WACKER sous la dénomination VP 1661, ou par la société DOW CORNING sous la dénomination 2501 cosmetic wax.
Les silicones les plus particulièrement préférées sont par exemple celles vendues par la société DOW CORNING sous la dénomination commerciale Q2-5220 et par la société RHONE POULENC sous la dénomination MIRASIL DMCO.

Le pinceau selon l'invention est particulièrement adapté pour le conditionnement et l'application de produit de vernis ou de soin des ongles comprenant un milieu aqueux. L'ensemble d'application de vernis ou de soin des ongles comprend un récipient muni d'un goulot et d'un bouchon solidaire d'un pinceau tel que défini précédemment, immergé, en position de stockage, dans un produit de vernis ou de soin des ongles comprenant un milieu aqueux contenu dans ce récipient.

Après conditionnement du pinceau sur le récipient, l'alcool polyvinylique présent à la surface des poils du pinceau se dissout facilement dans le produit aqueux de vernis ou de soin des ongles. Ce produit ainsi conditionné avec l'ensemble d'application contient donc, dans le milieu aqueux, l'alcool polyvinylique dissout qui peut être présent en une teneur allant de 0,005 % à 0,5 % en poids, par rapport au poids total du produit.

Les produits cosmétiques de vernis ou de soin des ongles comprennent généralement au moins un polymère filmogène, insoluble dans l'eau, sous forme de particules dispersées dans le milieu aqueux. Ce polymère filmogène étant insoluble dans l'eau, il est donc de nature chimique différente de l'alcool polyvinylique hydrosoluble employé pour traiter le pinceau défini précédemment.

Comme polymère filmogène, on peut employer de façon connue, les polymères les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats). Les polymères filmogènes de type radicalaires peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique.

Les esters de monomères acides peuvent être choisis parmi les esters de l'acide (méth)acrylique (encore appelé les (méth)acrylates), notamment des (méth)acrylates d'alkyle, en particulier d'alkyle en C₁-C₂₀, des (méth)acrylates d'aryle, en particulier d'aryle en C₆-C₁₀, des (méth)acrylates d'hydroxyalkyle, en particulier d'hydroxyalkyle en C₂-C₆.

Comme amides des monomères acides, on peut par exemple citer les (méth)acrylamides, et notamment les N-alkyl (méth)acrylamides, en particulier d'alkyl en C₂-C₁₂.

Les polymères vinyliques filmogènes peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Des polymères radicalaires utilisables dans les produits de vernis ou de soin des ongles sont notamment décrits dans les documents US-A-4158053, WO-A-97/42930, FR-A-2755009.

Parmi les polycondensats utilisables comme polymères filmogènes, on peut citer les polyuréthannes anioniques, cationiques, non-ioniques ou amphotères, les polyuréthannes-acryliques, les polyuréthannes-polyvinylpyrrolidones, les polyester-polyuréthannes, les polyéther-polyuréthannes, les polyurées, les polyurée/polyuréthannes, et leurs mélanges. Des polyuréthanes utilisables sont notamment décrits dans le document EP-A-680742

Parmi les polycondensats utilisables comme polymère filmogène, on peut également citer les polyesters, les polyesters amides, les polyesters à chaîne grasse, les polyamides, et les résines époxyesters. Des polyesters utilisables sont notamment décrits dans les documents US-A-3779993, ou bien encore vendus sous le nom Eastman AQ® par la société Eastman Chemical.

Les polymères d'origine naturelle, éventuellement modifiés, peuvent être choisis parmi la résine shellac, la gomme de sandaraque, les dammars, les élémis, les copals, les polymères cellulosiques insolubles dans l'eau, et leurs mélanges. De tels polymères sont notamment décrits dans la demande EP-A-676451.

La dispersion comprenant un ou plusieurs polymères filmogènes peut être préparée par l'homme du métier sur base de ses connaissances générales.

La taille des particules de polymères en dispersion aqueuse peut aller de 10 à 500 nm, et de préférence de 20 à 300 nm.

Le polymère en dispersion aqueuse peut être présent dans le produit de vernis ou de soin des ongles en une teneur allant de 1 % à 50 % en poids, de préférence de 5 % à 45% en poids de matière sèche de polymères filmogènes par rapport au poids total du produit.

Le produit de vernis ou de soin des ongles peut contenir des agents plastifiants et/ou des agents de coalescence qui sont bien connus de l'homme du métier.

L'invention consiste, mis à part les dispositions exposées ci-dessus, en un certain nombre d'autres dispositions dont il sera plus explicitement question après, à propos d'exemples de réalisations nullement limitatifs décrits en référence aux dessins annexés.

La figure 1 est une vue en élévation d'un pinceau de vernis à ongles selon l'invention.

La figure 2 est une vue en élévation d'un ensemble de vernis à ongles conforme à l'invention.

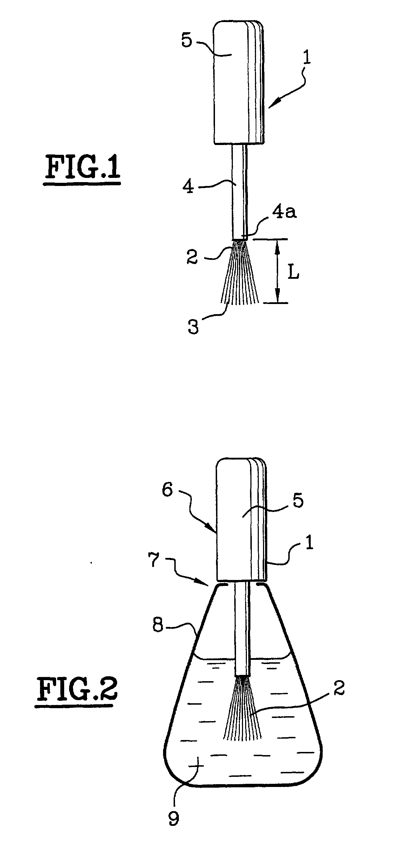

En se reportant à la figure 1, on peut voir un pinceau 1 pour l'application de vernis à ongles comprenant une touffe 2 de poils 3 fixés à une extrémité 4a d'une tige 4 et orientés sensiblement suivant la direction axiale de la tige. Un manchon cylindrique 5 est solidaire de l'extrémité de la tige 4 opposée à la touffe 2, et engagée dans ce manchon. Ce manchon cylindrique 5 sert d'organe de manoeuvre du pinceau ; il sert également de bouchon destiné, par exemple, à être vissé sur le goulot d'un flacon de vernis. La touffe 2 est obtenue à partir d'un faisceau de poils sensiblement parallèles replié en deux, environ à mi-longueur. La touffe 2 est fixée, de façon connue, à l'extrémité 4a de la tige 4 par engagement de la partie repliée de la touffe de poils dans un logement (non montré sur la figure 1) formé par un trou borgne s'ouvrant à l'extrémité de la tige 4. On peut se reporter aux demandes EP-A-651955 et EP-A-673612, dont le contenu est incorporé dans la présence demande à titre de référence, pour une description détaillée de la fixation de la touffe de poils sur la tige.

La figure 2 montre un ensemble 6 de vernis à ongles comportant le pinceau 1 de la figure 1 fixé sur le goulot 7 d'un flacon 8 contenant un produit 9 de vernis à ongles comprenant de l'eau, la touffe 2 de poils immergeant dans le produit 9. Le produit 9 comprend l'alcool polyvinylique selon l'invention dissout dans le milieu aqueux ; il comprend en outre un polymère filmogène, insoluble dans l'eau, sous forme de particules dispersées dans le milieu aqueux.

On donne ci-après un exemple de traitement de pinceau selon le procédé de l'invention et des exemples comparatifs réalisés avec un procédé ne faisant pas partie de l'invention.

### Exemple 1 :

On a préparé une solution renforcatrice Si ayant la composition suivante :

### Solution 1 :

- Alcool polyvinylique partiellement acétylé (AIRVOL 205 de la société AIR PRODUCTS CHEMICAL) 1,5 g
- Alcool éthylique 66 g
- Eau qsp 100 g

On a traité avec cette solution un pinceau ayant les caractéristiques suivantes :
longueur apparente des poils : 18 mm
90 % de poils cylindriques en polyamide 11 (RILSAN), 15/100 mm, chargés à 5 % de bisulfure de molybdène, environ 110 poils
10 % de poils cylindriques en polyamide 6-12 (Nylon TYNEX), 8/100 mm, environ 46 poils.

On a plongé la touffe du pinceau à mi-longueur dans la solution pendant 1 seconde.

Puis on a retiré le pinceau et laissé sécher à l'air. Après séchage, le dépôt d'alcool polyvinylique en surface des poils n'est pas collant et confère aux poils une bonne souplesse tout en les maintenant en une touffe uniforme.

Lors de l'immersion du pinceau dans un produit aqueuse de vernis à ongles, par exemple identique à celles des exemples de la demande EP-A-648485, le polymère en surface des poils se dissout très bien dans le milieu aqueux et ne modifie pas les propriétés cosmétiques du vernis. La touffe homogène du pinceau permet une application d'une couche uniforme du vernis sur l'ongle.

On a disposé le pinceau de l'exemple 1 dans un flacon contenant le produit de vernis à ongles suivant :
- Dispersion aqueuse de polyester-polyuréthane à 35 % de matières sèches (AVALURE UR 405 de GOODRICH) 25 g MA
- acétyl citrate de tributyle 2,5 g
- éthanol 5 g
- argile 1 g
- pigments 3 g
- conservateurs qs
- eau qsp 100 g

Après immersion de la touffe du pinceau dans le produit de vernis à ongles, l'alcool polyvinylique se dissout bien dans le vernis. Ce dernier ne se déstabilise pas et conserve ses propriétés cosmétiques.

### Exemple 2 :

On a procédé de la même façon que celle décrite à l'exemple 1 en employant la solution renforcatrice 2 suivante :

### Solution 2 :

- alcool polyvinylique partiellement acétylé (AIRVOL 540 de AIR PRODUCTS CHEMICAL) 1,7 g
- éthanol 50 g
- eau qsp 100 g

Après immersion de la touffe du pinceau dans le produit de vernis à ongles, l'alcool polyvinylique se dissout bien dans le vernis. Ce dernier ne se déstabilise pas et conserve ses propriétés cosmétiques.

### Exemples 3 et 4 comparatifs :

On a réalisé 2 solutions renforcatrices, ne faisant pas partie de l'invention, ayant la composition suivante :

### Solution 3 :

- polyméthacrylate de sodium (DARVAN 7 de VANDERBILT) 2,5 g
- éthanol 20 g
- eau qsp 100g

### Solution 4 :

- Hydroxypropyl méthylcellulose (METHOCEL E4 M UG de DOW CHEMICAL) 0,67 g
- éthanol 66,6 g
- eau qsp 100 g

Pour chaque solution, on a traité un pinceau ayant les mêmes caractéristiques que celui de l'exemple 1 en le trempant à mi-longueur pendant 1 seconde dans la solution.

On a constaté que le traitement avec la solution 3 ne permet pas d'obtenir un séchage rapide de la touffe de poils imprégnée puisque la touffe n'est pas sèche au toucher au bout de 30 minutes. Ce temps de séchage trop long ne convient pas pour une mise en oeuvre industrielle du traitement du pinceau.

La solution 4 conduit, après trempage et séchage, à la formation d'un film dur. De plus, le film se casse facilement lorsqu'il est soumis à un faible choc (par exemple torsion de la touffe) et il s'ensuit que les poils ne sont plus renforcés et la touffe peut alors se déformer facilement.

Seul un pinceau traité conforme à l'exemple 1 ou 2 selon l'invention permet d'obtenir un bon renfort de la touffe ainsi qu'une bonne dissolution de l'alcool polyvinylique dans le produit aqueux de vernis sans nuire à la stabilité, ni aux propriétés cosmétiques du vernis.

## Revendications

1. Pinceau pour l'application d'un produit de maquillage ou de soin, comportant une touffe (2) de poils (3) sensiblement parallèles, une première extrémité de ces poils (3) étant fixé à une extrémité (4a) libre d'une tige (4), **caractérisé par** le fait que les poils (3) de la touffe (2) sont imprégnés en surface d'un alcool polyvinylique partiellement acétylé comportant au moins des unités de formule (I) et (II) : 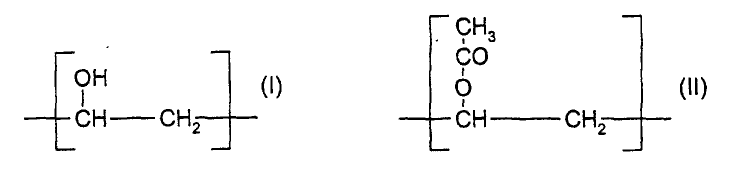 dans lesquelles les unités de formules (II) sont présentes dans des proportions allant de 3 % à 40 % en mole par rapport au polymère, ledit alcool polyvinylique ayant un poids moléculaire moyen en poids allant de 10.000 à 190.000.

2. Pinceau selon la revendication 1, **caractérisé par** le fait que l'alcool polyvinylique a un poids moléculaire moyen en poids allant de 10.000 à 120.000, et mieux de 31.000 à 50.000.

3. Pinceau selon l'une quelconque des revendications 1 ou 2, **caractérisé par** le fait que les unités de formules (II) sont présentes en une proportion allant de 10 à 15 % en mole.

4. Pinceau selon l'une quelconque des revendications précédentes, **caractérisée par** le fait que l'alcool polyvinylique consiste essentiellement en des unités de formule (I) et (II).

5. Pinceau selon l'une quelconque des revendications précédentes, **caractérisé par** le fait que les poils sont en un matérieu choisi dans le groupe formé parmi les polyamides, les polyesters, les polyéther-bloc-amides, le polyéthylène, le polytétrafluoroéthylène, le polyfluorure de vinylidène, les polychlorures de vinyle, la viscose, la rayonne, les polyacétals, les soies naturelles, ou leurs mélanges.

6. Pinceau selon l'une quelconque des revendications précédentes, **caractérisée par** le fait que les poils ont une section inscrite dans un cercle (φ) de diamètre allant de 4 centièmes à 40 centièmes de millimètre.

7. Pinceau selon l'une quelconque des revendications précédentes, **caractérisé par** le fait que la touffe (2) de poils (3) a une longueur (L) allant de 5 à 25 millimètres.

8. Pinceau selon l'une quelconque des revendications précédentes, **caractérisé par** le fait que le nombre des poils (3) constituant la touffe (2) va de 100 à 600.

9. Pinceau pour vernis à ongles, **caractérisé en ce qu'**il consiste en un pinceau conforme à l'une quelconque des revendications précédentes.

10. Procédé de gainage de poils d'une touffe (2) de poils (3) d'un pinceau (1) comportant une touffe de poils sensiblement parallèles, une première extrémité de ces poils étant fixée à une extrémité libre d'une tige, **caractérisé par** le fait que l'on met en contact au moins une partie de la touffe de poils avec une composition aqueuse d'un alcool polyvinylique partiellement acétylé comportant au moins des unités de formule (I) et (II) : 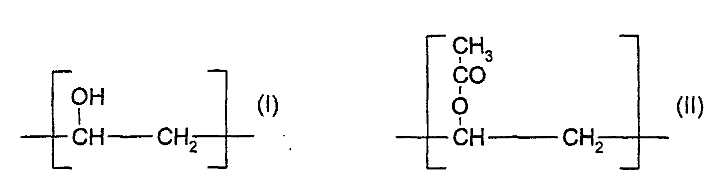 dans lesquelles les unités de formules (II) sont présentes dans des proportions allant de 3 % à 40 % en mole par rapport au polymère, ledit alcool polyvinylique ayant un poids moléculaire moyen en poids allant de 10.000 à 190.000, puis on retire la touffe de poils de la composition et on la laisse sécher.

11. Procédé selon la revendication 10, **caractérisé par** le fait que l'alcool polyvinylique a un poids moléculaire moyen en poids allant de 10.000 à 120.000, et mieux de 31.000 à 50.000.

12. Procédé selon l'une des revendications 10 ou 11, **caractérisé par** le fait que les unités de formules (II) sont présentes en une proportion allant de 10 à 15 % en mole.

13. Procédé selon l'une quelconque des revendications 10 à 12, **caractérisé par** le fait que l'alcool polyvinylique est présent dans la composition en une teneur allant de 0,1 % à 10 % en poids, par rapport au poids total de la composition, et mieux de 0,5 % à 5 % en poids.

14. Procédé selon l'une quelconque des revendications 10 à 13, **caractérisé par** le fait que la composition comprend en outre au moins un alcool inférieur en C₂-C₅.

15. Procédé selon la revendication 14, **caractérisé par** le fait que l'alcool inférieur est l'éthanol.

16. Procédé selon l'une quelconque des revendications 10 à 15, **caractérisé par** le fait que l'alcool inférieur est présent en une teneur allant de 0 % à 80 % en poids, par rapport au poids total de la composition, et mieux de 40 % à 70 % en poids.

17. Procédé selon l'une quelconque des revendications 10 à 16, **caractérisé par** le fait que la composition comprend en outre un agent de mouillage.

18. Procédé selon la revendication 17, **caractérisé par** le fait que l'agent de mouillage est un diméthicone copolyol.

19. Ensemble d'application (6) de produit de vernis ou de soin des ongles, constitué d'un récipient (8) muni d'un goulot (7) et d'un bouchon (5) solidaire d'un pinceau (1) immergé en position de stockage, dans un produit (9) de vernis ou de soin des ongles, contenu dans ce récipient, **caractérisé par** le fait que le produit comprend un milieu aqueux et que le pinceau est conforme à l'une quelconque des revendications 1 à 9.

20. Ensemble d'application (6) de vernis ou de soin des ongles, constitué d'un récipient (8) muni d'un goulot (7) et d'un bouchon (5) solidaire d'un pinceau (1) immergé, en position de stockage, dans un produit (9) de vernis ou de soin des ongles contenu dans ce récipient, **caractérisé par** le fait que le produit comprend un milieu aqueux contenant au moins un alcool polyvinylique partiellement acétylé comportant au moins des unités de formule (I) et (II) : 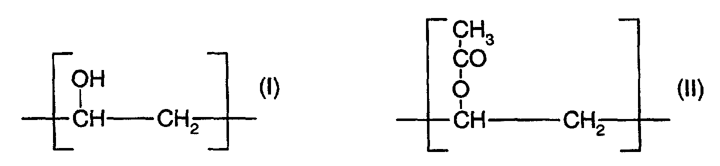 dans lesquelles les unités de formules (II) sont présentes dans des proportions allant de 3 % à 40 % en mole par rapport au polymère, ledit alcool polyvinylique ayant un poids moléculaire moyen en poids allant de 10.000 à 190.000.

21. Ensemble selon la revendication 20, **caractérisé par** le fait que l'alcool polyvinylique a un poids moléculaire moyen en poids allant de 10.000 à 120.000, et mieux de 31.000 à 50.000.

22. Ensemble selon la revendication 20 ou 21, **caractérisé par** le fait que les unités de formules (II) sont présentes en une proportion allant de 10 à 15 % en mole par rapport au polymère.

23. Ensemble selon l'une quelconque des revendications 20 à 22, **caractérisée par** le fait que l'alcool polyvinylique consiste essentiellement en des unités de formule (I) et (II).

24. Ensemble selon l'une quelconque des revendications 20 à 23, **caractérisé par** le fait que ledit alcool polyvinylique est présent dans le produit en une teneur allant de 0,005 % à 0,5 % en poids, par rapport au poids total du produit.

25. Ensemble selon l'une quelconque des revendications 19 à 24, **caractérisé par** le fait que le produit comprend au moins un polymère filmogène, insoluble dans l'eau, sous forme de particules dispersées dans le milieu aqueux.

## Patentansprüche

1. Pinsel zum Auftragen eines Produktes zum Schminken oder zu Pflege, der ein Büschel (2) von im wesentlichen parallelen Haaren (3) aufweist, wobei ein Ende der Haare (3) am freien Ende (4a) eines Schaftes (4) befestigt ist, **dadurch gekennzeichnet, daß** die Haare (3) des Büschels (2) an der Oberfläche mit einem teilweise acetylierten Polyvinylalkohol imprägniert sind, der zumindest Einheiten der Formeln (I) und (II) aufweist: 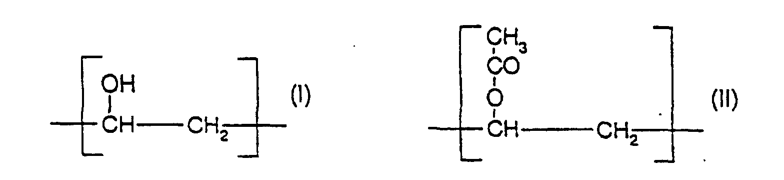 worin die Einheiten der Formel (II) in Mengenanteilen von 3 bis 40 Mol-%, bezogen auf das Polymer, vorliegen, wobei der Polyvinylalkohol ein Gewichtsmittel des Molekulargewichts von 10.000 bis 190 000 aufweist.

2. Pinsel nach Anspruch 1, **dadurch gekennzeichnet, daß** der Polyvinylalkohol ein Gewichtsmittel des Molekulargewichts von 10 000 bis 120 000 und besser noch 31 000 bis 50 000 aufweist.

3. Pinsel nach einem der Ansprüche 1 oder 2, **dadurch gekennzeichnet, daß** die Einheiten der Formel (II) in Mengenanteilen von 10 bis 15 Mol-% vorliegen.

4. Pinsel nach einem der vorhergehenden Ansprüche, **dadurch gekennzeichnet, daß** der Polyvinylalkohol im wesentlichen aus Einheiten (I) und (II) besteht.

5. Pinsel nach einem der vorhergehenden Ansprüche, **dadurch gekennzeichnet, daß** die Haare aus einem Material bestehen, das unter den Polyamiden, Polyestern, Polyetherblockamiden, Polyethylen, Polytetrafluorethylen, Polyvinylidenfluorid, Polyvinylchloriden, Viscose, Rayon, Polyacetalen, natürlicher Seide oder deren Gemischen ausgewählt ist.

6. Pinsel nach einem der vorhergehenden Ansprüche, **dadurch gekennzeichnet, daß** die Haare einen Querschnitt aufweisen, der in einen Kreis mit einem Durchmesser (φ) von 4/100 bis 40/100 mm einbeschrieben werden kann.

7. Pinsel nach einem der vorhergehenden Ansprüche, **dadurch gekennzeichnet, daß** das Büschel (2) der Haare (3) eine Länge (L) von 5 bis 25 mm aufweist.

8. Pinsel nach einem der vorhergehenden Ansprüche, **dadurch gekennzeichnet, daß** die Anzahl der Haare (3), die das Büschel (2) bilden, im Bereich von 100 bis 600 liegt.

9. Pinsel für Nagellacke, **dadurch gekennzeichnet, daß** es sich um einen Pinsel nach einem der vorhergehenden Ansprüche handelt.

10. Verfahren zum Umhüllen eines Büschels (2) von Haaren (3) eines Pinsels (1), der ein Büschel von im wesentlichen parallelen Haaren aufweist, wobei ein Ende der Haare am freien Ende eines Schaftes befestigt ist, **dadurch gekennzeichnet, daß** mindestens ein Teil des Haarbüschels mit einer wäßrigen Zusammensetzung eines teilweise acetylierten Polyvinylalkohol in Kontakt gebracht wird, der zumindest Einheiten der Formeln (I) und (II) aufweist: 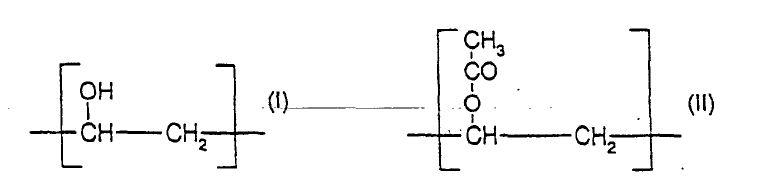 worin die Einheiten der Formel (II) in Mengenanteilen von 3 bis 40 Mol-%, bezogen auf das Polymer, vorliegen, wobei der Polyvinylalkohol ein Gewichtsmittel des Molekulargewichts von 10 000 bis 190 000 aufweist,
und das Haarbüschel aus der Zusammensetzung genommen und trocknen gelassen wird.

11. Verfahren nach Anspruch 10, **dadurch gekennzeichnet, daß** der Polyvinylalkohol ein Gewichtsmittel des Molekulargewichts von 10 000 bis 120 000 und besser noch 31 000 bis 50 000 aufweist.

12. Verfahren nach einem der Ansprüche 10 oder 11, **dadurch gekennzeichnet, daß** die Einheiten der Formel (II) in Mengenanteilen von 10 bis 15 Mol-% vorliegen.

13. Verfahren nach einem der Ansprüche 10 bis 12, **dadurch gekennzeichnet, daß** der Polyvinylalkohol in der Zusammensetzung in Mengenanteilen von 0,1 bis 10 Gew.-%, bezogen auf das Gesamtgewicht der Zusammensetzung, und besser 0,5 bis 5 Gew.-% vorliegt.

14. Verfahren nach einem der Ansprüche 10 bis 13, **dadurch gekennzeichnet, daß** die Zusammensetzung ferner mindestens einen niederen C₂₋₅-Alkohol enthält.

15. Verfahren nach Anspruch 14, **dadurch gekennzeichnet, daß** der niedere Alkohol Ethanol ist.

16. Verfahren nach einem der Ansprüche 10 bis 15, **dadurch gekennzeichnet, daß** der niedere Alkohol in Mengenanteilen von 0 bis 80 Gew.-%, bezogen auf das Gesamtgewicht der Zusammensetzung, und besser 40 bis 70 Gew.-% vorliegt.

17. Verfahren nach einem der Ansprüche 10 bis 16, **dadurch gekennzeichnet, daß** die Zusammensetzung ferner ein Netzmittel enthält.

18. Verfahren nach Anspruch 17, **dadurch gekennzeichnet, daß** das Netzmittel ein Dimethiconcopolyol ist.

19. Behältnis (6) zum Auftragen von dekorativen oder pflegenden Nagellacken, das aus einem Behälter (8) mit einem Hals (7) und einem Verschluß (5) besteht, der mit einem Pinsel (1) fest verbunden ist, der bei der Aufbewahrung in einen dekorativen oder pflegenden Nagellack (9), der in dem Behälter (8) enthalten ist, eintaucht, **dadurch gekennzeichnet, daß** der Lack ein wäßriges Medium enthält und der Pinsel ein Pinsel nach einem der Ansprüche 1 bis 9 ist.

20. Behältnis (6) zum Auftragen von dekorativen oder pflegenden Nagellacken, das aus einem Behälter (8) mit einem Hals (7) und einem Verschluß (5) besteht, der mit einem Pinsel (1) fest verbunden ist, der bei der Aufbewahrung in einen dekorativen oder pflegenden Nagellack (9), der in dem Behälter (8) enthalten ist, eintaucht, **dadurch gekennzeichnet, daß** der Lack ein wäßriges Medium mit mindestens einem teilweise acetyliertem Polyvinylalkohol enthält, der zumindest Einheiten der Formeln (I) und (II) aufweist: 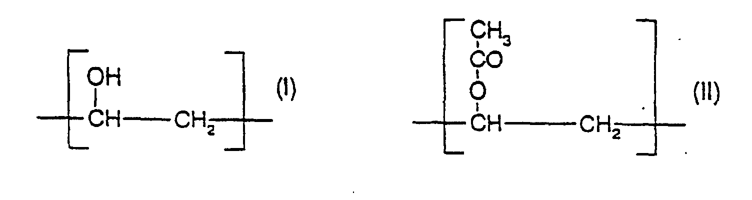 worin die Einheiten der Formel (II) in Mengenanteilen von 3 bis 40 Mol-%, bezogen auf das Polymer, vorliegen, wobei der Polyvinylalkohol ein Gewichtsmittel des Molekulargewichts von 10 000 bis 190 000 aufweist.

21. Behältnis nach Anspruch 20, **dadurch gekennzeichnet, daß** der Polyvinylalkohol ein Gewichtsmittel des Molekulargewichts von 10 000 bis 120 000 und besser 31 000 bis 50 000 aufweist.

22. Behältnis nach Anspruch 20 oder 21, **dadurch gekennzeichnet, daß** die Einheiten der Formel (II) in Mengenanteilen von 10 bis 15 Mol-%, bezogen auf das Polymer, vorliegen.

23. Behältnis nach einem der Ansprüche 20 bis 22, **dadurch gekennzeichnet, daß** der Polyvinylalkohol im wesentlichen aus Einheiten (I) und (II) besteht.

24. Behältnis nach einem der Ansprüche 20 bis 23, **dadurch gekennzeichnet, daß** der Polyvinylalkohol in dem Lack in einer Menge von 0,005 bis 0,5 %, bezogen auf das Gesamtgewicht des Lackes, vorliegt.

25. Behältnis nach einem der Ansprüche 19 bis 24, **dadurch gekennzeichnet, daß** der Lack mindestens ein in Wasser unlösliches, filmbildendes Polymer in Form von in dem wäßrigen Medium dispergierten Partikeln enthält.

## Claims

1. Brush for the application of a make-up or beauty product, comprising a tuft (2) of approximately parallel bristles (3), a first end of these bristles (3) being fastened to a free end (4a) of a wand (4), **characterized in that** the bristles (3) of the tuft (2) are surface-impregnated with a partially acetylated polyvinyl alcohol comprising at least units of formulae (I) and (II): 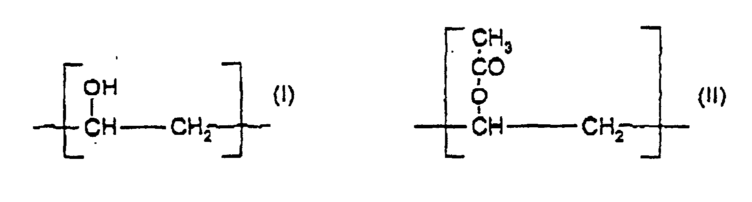 in which the units of formula (II) are present in proportions ranging from 3 mol% to 40 mol% with respect to the polymer, the said polyvinyl alcohol having a weight-average molecular weight ranging from 10,000 to 190,000.

2. Brush according to Claim 1, **characterized in that** the polyvinyl alcohol has a weight-average molecular weight ranging from 10,000 to 120,000 and even better from 31,000 to 50,000.

3. Brush according to either of Claims 1 and 2, **characterized in that** the units of formula (II) are present in a proportion ranging from 10 to 15 mol%.

4. Brush according to any one of the preceding claims, **characterized in that** polyvinyl alcohol essentially consists of units of formulae (I) and (II).

5. Brush according to any one of the preceding claims, **characterized in that** the bristles are made of a material chosen from the group formed from polyamides, polyesters, polyether-block-amides, polyethylene, polytetrafluoroethylene, polyvinylidene fluoride, polyvinyl chlorides, viscose, rayon, polyacetals, natural silks, or blends thereof.

6. Brush according to any one of the preceding claims, **characterized in that** the bristles have a cross section inscribed in a circle (φ) of diameter ranging from 4/100 to 40/100 of a millimetre.

7. Brush according to any one of the preceding claims, **characterized in that** the tuft (2) of bristles (3) has a length (L) ranging from 5 to 25 millimetres.

8. Brush according to any one of the preceding claims, **characterized in that** the number of bristles (3) making up the tuft (2) ranges from 100 to 600.

9. Nail-varnish brush, **characterized in that** it consists of a brush in accordance with any one of the preceding claims.

10. Method of coating bristles of a tuft (2) of bristles (3) of a brush (1), comprising a tuft of approximately parallel bristles, a first end of these bristles being fastened to a free end of a wand, **characterized in that** at least part of the tuft of bristles is brought into contact with an aqueous composition of a partially acetylated polyvinyl alcohol comprising at least units of formulae (I) and (II): 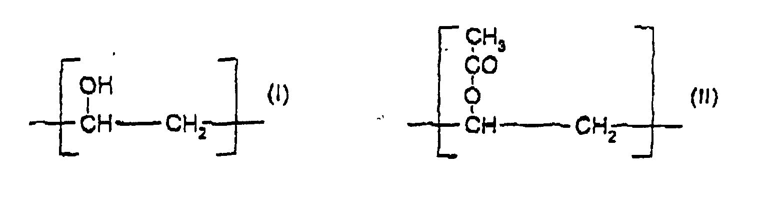 in which the units of formula (II) are present in proportions ranging from 3 mol% to 40 mol% with respect to the polymer, the said polyvinyl alcohol having a weight-average molecular weight ranging from 10,000 to 190,000, and then the tuft of bristles is removed from the composition and left to dry.

11. Method according to Claim 10, **characterized in that** the polyvinyl alcohol has a weight-average molecular weight ranging from 10,000 to 120,000 and even better from 31,000 to 50,000.

12. Method according to either of Claims 10 and 11, **characterized in that** the units of formula (II) are present in a proportion ranging from 10 to 15 mol%.

13. Method according to any one of Claims 10 to 12, **characterized in that** the polyvinyl alcohol is present in the composition in an amount ranging from 0.1% to 10% by weight, and even better from 0.5% to 5% by weight, with respect to the total weight of the composition.

14. Method according to any one of Claims 10 to 13, **characterized in that** the composition furthermore includes at least one C₂-C₅ lower alcohol.

15. Method according to Claim 14, **characterized in that** the lower alcohol is ethanol.

16. Method according to any one of Claims 10 to 15, **characterized in that** the lower alcohol is present in an amount ranging from 0% to 80% by weight, and even better from 40% to 70% by weight, with respect to the total weight of the composition.

17. Method according to any one of Claims 10 to 16, **characterized in that** the composition furthermore includes a wetting agent.

18. Method according to Claim 17, **characterized in that** the wetting agent is a dimethicone copolyol.

19. Application assembly (6) for a nail-varnish or nail-care product, consisting of a container (8) provided with a neck (7) and with a stopper (5) integral with a brush (1) which is immersed, in a storage position, in a nail-varnish or nail-care product (9) contained in this container, **characterized in that** the product includes an aqueous medium and in that the brush is in accordance with any one of Claims 1 to 9.

20. Application assembly (6) for a nail- varnish or nail-care product, consisting of a container (8) provided with a neck (7) and with a stopper (5) integral with a brush (1) which is immersed, in a storage position, in a nail-varnish or nail-care product (9) contained in this container, **characterized in that** the product includes an aqueous medium containing at least one partially acetylated polyminyl alcohol comprising at least units of formulae (I) and (II): 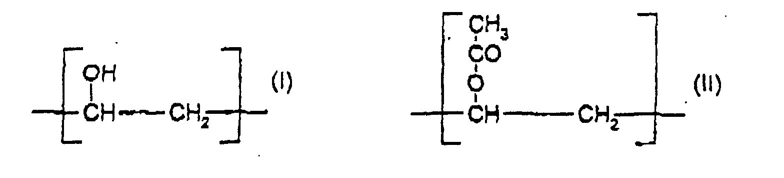 in which the units of formula (II) are present in proportions ranging from 3 mol% to 40 mol% with respect to the polymer, the said polyvinyl alcohol having a weight-average molecular weight ranging from 10,000 to 190,000.

21. Assembly according to Claim 20, **characterized in that** the polyvinyl alcohol has a weight-average molecular weight ranging from 10,000 to 120,000 and even better from 31,000 to 50,000.

22. Assembly according to Claim 20 or 21, **characterized in that** the units of formula (II) are present in a proportion ranging from 10 to 15 mol% with respect to the polymer.

23. Assembly according to any one of Claims 20 to 22, **characterized in that** the polyvinyl alcohol essentially consists of units of formulae (I) and (II).

24. Assembly according to any one of Claims 20 to 23, **characterized in that** the said polyvinyl alcohol is present in the product in an amount ranging from 0.005% to 0.5% by weight with respect to the total weight of the product.

25. Assembly according to any one of Claims 19 to 24, **characterized in that** the product furthermore includes a water-insoluble film-forming polymer in the form of particles dispersed in the aqueous medium.
